# EUROPEAN PATENT SPECIFICATION

(11) **EP 1 786 314 B1**
(45) Date of publication and mention of the grant of the patent: **08.04.2015**
(21) Application number: 05785108.1
(22) Date of filing: 06.07.2005
(51) Int. Cl.: A61B 3/135, A61B 3/14

(54) **REFLECTION MICROSCOPE FOR EXAMINATION OF THE CORNEAL ENDOTHELIUM AND METHOD OF OPERATING SAME**
REFLEXIONSMIKROSKOP ZUR UNTERSUCHUNG DES ENDOTHELIUMS DER KORNEA UND VERFAHREN ZUR VERWENDUNG DIESES MIKROSKOPS
MICROSCOPE EN RÉFLEXION POUR L'EXAMEN DE L'ENDOTHELIUM CORNÉEN

(30) Priority: 08.07.2004 EP 04425498
(43) Date of publication of application: 23.05.2007
(73) Proprietor: Costruzioni Strumenti Oftalmici C.S.O. S.r.l., 50018 Scandicci FI (IT)
(72) Inventor: MURA, Sergio, I-50018 Scandicci - Firenze (IT)
(74) Representative: Soldatini, Andrea
(86) International application number: PCT/IB2005/001908
(87) International publication number: WO 2006/006048

(56) References cited:
- EP-A- 0 628 281
- EP-A- 0 641 541
- WO-A-03/015623
- US-A- 5 381 194
- US-A- 6 164 778

## Description

### Field of the invention

The present invention refers to a new non-contact endothelium reflection microscope apparatus that permits to automatically obtain the endothelium image and to display clinically useful parameters such as the number and density of the cells, shape, surface, minimum, maximum and medium area, standard deviation, variation coefficient, percentage of cells of various shapes, area distribution histogram, perimeter distribution histogram.

### Background of the invention

The endothelium is the most internal layer of the tissues forming the cornea, and consists of a single layer of flat polygonal cells. The endothelium function is to adjust the water contents, permitting a suitable hydration of the cornea. The shape and amount of the cells influence the quality of the vision. The cornea transparency is subjected to a very delicate balance, and a number of diseases can produce a loss of the transparency.

The endothelium cells are of hexagonal shape in the children and in the young people. They do not reproduce themselves and. At birth, the density is about 4000 cells per square millimeter but as years pass the number decreases and the cells change their shape. The average density in an adult becomes of 2700 cells per square millimeter, in a range from 1600 to 3200 cells for square millimeter. The loss of cells brings about two main morphologic changes: the presence of cells with different surface area, and the increase in the amount of cells shaped differently from the basic hexagonal shape.

The evaluation of the cornea endothelium is useful to have a first clinic indication regarding the risks of a surgical step, and for checking a diagnostic assumption or a therapy effectiveness. In this kind of evaluation, it is very important to observe heterogeneous parts, such as intracellular and intercellular areas of no reflectance (dark spots), hyper reflective areas (bright spots), empty areas in the cells layer (guttae), bubbles, Descemet's membrane rupture lines.

Said parts can be checked in relationship with the evolution of different endothelium diseases of inflammatory or dystrophic nature. The quantity evaluation permits to assign to a determined photographic field a numeric parameter useful for the study of the endothelium variations in time, or for the comparison among different patients.

The most easily accessible parameter is the average cellular density, obtained for comparison or and by counting the cellular elements. The first method is carried out by comparing the cellular dimensions with the dimensions of the hexagonal reticules that correspond to determined densities. The counting of the cellular elements, instead, is carried out by using fixed or variable reticules.

The two methods give no information on the evolution of the cellular dimensions. This can be obtained by identifying, above and beyond the dimension of the average cellular area and its variability, also the perimeters of the cells. The endothelium reflection microscopic observation was first introduced in the ophthalmologic practice around 1960 by David Maurice who, by modifying a metallography microscope, was able to obtain photographic images of a rabbit corneal endothelium. Exploiting the same theoretic principles, a microscope was subsequently proposed capable of taking photographs of the endothelium without contacting the eye.

The non-contact reflection microscope apparatus are generally derived from normal slit lamps with a high magnification microscope. The technical principle on which they are based is the visualization of a determined structure in relation to its capability of reflecting an incident ray of light used for the illumination. In the commonly used technique (triangulation), the observation angle is of about 45°, the microscope being placed such that the bisector axis of the angle of view is perpendicular to the plane tangent to the corneal surface.

The non-contact endothelium microscopy is particular indicated in all cases where the contact with the cornea can be dangerous, and therefore immediately after surgery or when there is an extreme structural fragility of the cornea. With the integration of the microscope with techniques of image analysis, the apparatus is able to give also a quantitative description of the endothelium tissue, expressed by the average cellular density and specific morphometric parameters.

A non-contact endothelium microscope according to the prior art is shown for example in European Patent Application n. EP628281. The optical unit in this apparatus comprises an illuminating system, for obliquely illuminating through a slit an eyeball surface of a subject eye, and an eye-front observation optical system in which alignment-use indicator light for positional adjustment of the imaging optical axis is projected towards the eye and the resulting reflected light is received and imaged by a TV camera. An enlarged-imaging optical system is also provided for enlarged observation or enlarged photographing of the subject part by the TV camera based on slit illuminating light with which the eyeball surface has been illuminated.

A photo-detector is arranged so as to detect a position at which the enlarged-imaging optical system has been focused on the subject part, via a reflected optical path other than that via which the enlarged image has been formed by the enlarged-imaging optical system. The whole optical unit is automatically moved both in a transversal direction and in a direction toward the eye, in response to the location of the above mentioned indicator light as displayed on a screen of a video monitor, so that the location chases a specified position on the screen. The enlarged visual image of the subject portion of the cornea is thus photographed via the TV camera when the photo-detector detects the focusing.

The above described system, with the use of a focusing detection photo-detector placed along a supplementary reflected optical path, renders the apparatus sophisticated, and thus costly to be produced and maintained in order to have reliable results.

### Summary of the invention

The apparatus according to the invention permits to have the endothelium test without use of sensors, photosensors or other devices placed onto a reflected optical path. A higher quality endothelium image is obtained with a reduced use of electronic components and so with greater reliability, completeness and use flexibility in comparison with the prior art.

The essential characteristics of the microscope apparatus for the morphometric analysis of the cornea endothelium with direct image acquisition according to the invention are defined by the first of the annexed claims.

### Brief description of the drawings

The characteristics and advantages of the microscope apparatus for the morphometric analysis of the cornea endothelium with direct image acquisition according to the present invention will be made clearer by the following description of embodiments thereof, given purely as a an example and not limitative, with reference to the accompanying drawings, wherein:
- figure 1 is an optical path diagram of a first embodiment of the apparatus according to the invention;
- figure. 2 is an optical path diagram of a second embodiment of the apparatus according to the invention;
- figure 3 is a block diagram representing the hardware configuration of an apparatus according to the invention;
- figures 4 and 5 are explanatory views showing respective images displayed on a monitor screen during the image acquisition procedures according to the invention;
- figure 6 schematically represents exemplifying reflections obtained with the apparatus; and
- figures 7 to 9 are flowcharts showing the procedures for image acquisition with the apparatus according to the invention.

### Detailed description of the Invention

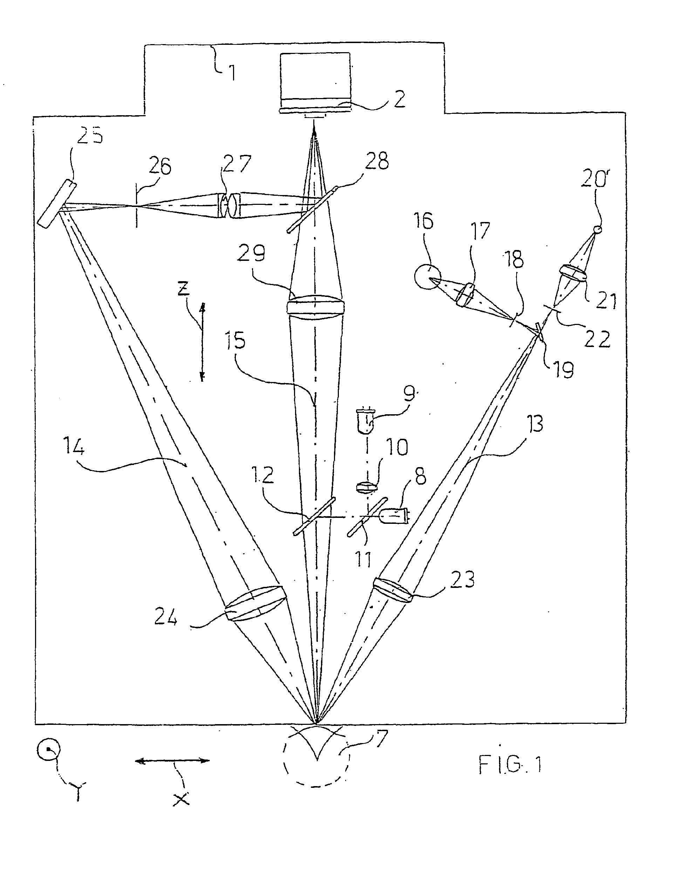

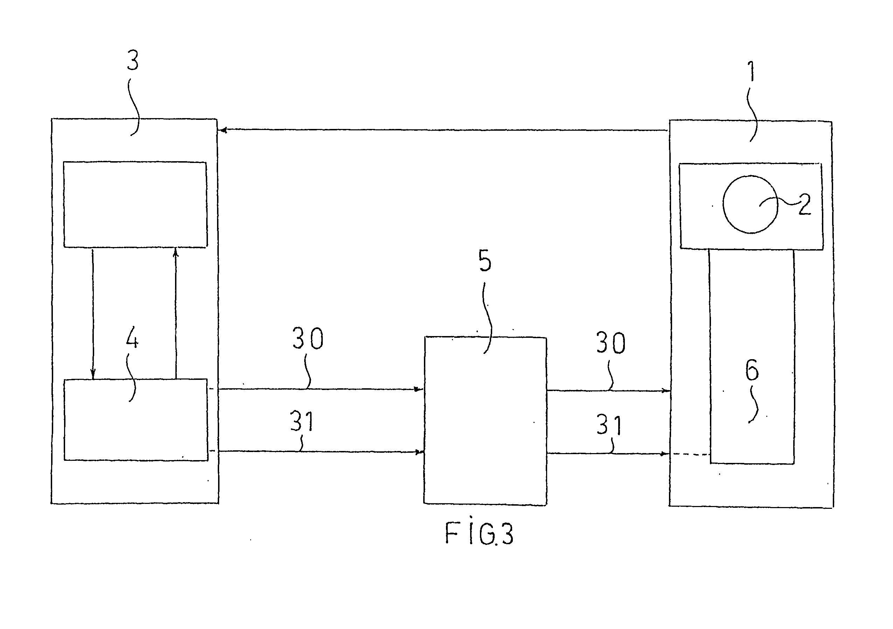

Referring to figures 1 to 3, the apparatus according to the invention comprises a movable optical head or microscope 1 provided with a CCD high speed camera 2, i.e. a monochrome digital camera with shooting capacity of at least one hundred frames per second with FireWire high speed data output, i.e. with IEEE 1394 port or equivalent.

The high speed camera 2 is directly connected to a CPU unit 3. The unit 3 comprises a controller 4, e.g. a 65XX type controller produced by the company National Instruments (United States, Texas) or equivalent. The controller 4 controls a power driver board 5, so that the signal coming from the CPU unit 3 is suitable for driving electric DC motors 6 as described hereinafter.

The function of the motors 6 is to set in position the microscope 1 with the camera 2, following to automatic control by the CPU unit 3 so that the eye center 7 to be examined is found. Such a finding is obtained via a reflection onto the cornea surface of the light emitted by an infrared LED 8 mounted onto the mobile head of the apparatus, consisting of the microscope 1 with the camera 2.

The cited electronic components are connected each other according to known configurations. Considering instead more in detail the optical scheme of figure 1, a second LED. 9 with associated optics 10 is arranged nearby the infrared LED 8 for providing the fixation point in association with a semireflecting mirror 11 and a semireflecting mirror 12, necessary to arrange the microscope in a way to center the patient eye and to obtain the triangulation necessary for the test. These components, like the other that follow and that form the optical scheme, are triangulation elements for the endothelium test, known and already in use for this kind of applications.

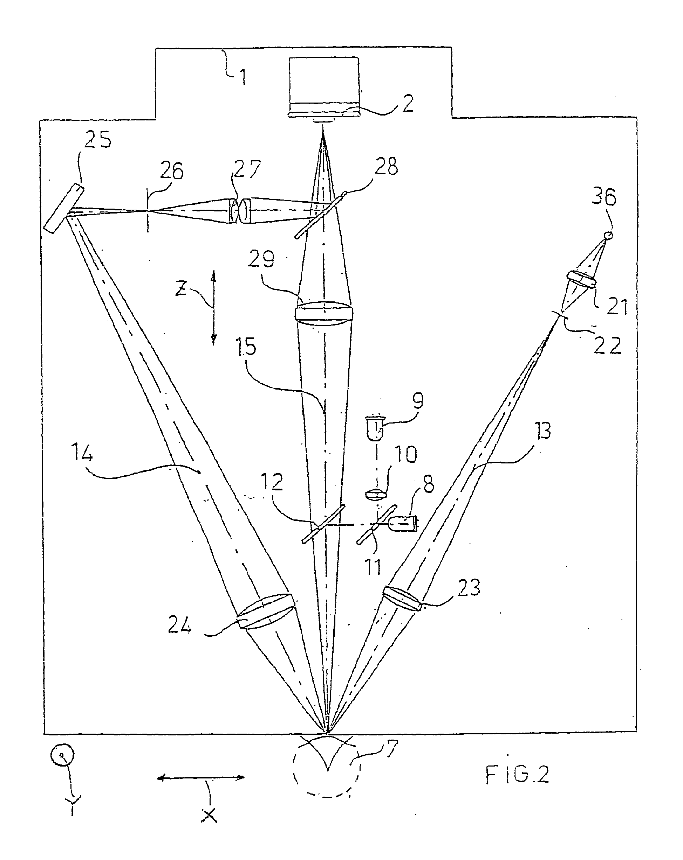

The optical scheme comprises then a side projection axis 13, a side reflection axis 14 and a central channel 15. In the embodiment of figure 1, transversally to the side projection axis 13, a halogen lamp 16 is arranged with a lamp condenser 17 and a slit 18. Along the side projection axis 13 there is also placed a semireflecting mirror 19 receiving the light beam generated by the halogen lamp 16 and the beam that can be generated by a photoflash 20 located at the start of the side projection axis 13. On the same axis, the photoflash 20 is followed by a photoflash condenser 21, a slit 22 and, beyond the mirror 19, by a an optical unit 23 that concentrates the beam onto the patient eye 7. In the embodiment of figure 2 the lamp 16, the condenser 17, the slit 18, the semireflecting mirror 19 and the photoflash 20 are replaced by a stroboscopic lamp 36 activated analogously and with the same function to the previous elements.

Along the side reflection axis 14 there is arranged a side reflection optical unit 24 that concentrates the reflected beam and the endothelium image to a mirror 25, from which the beam and the image signal are reflected to the central channel 15 passing through a filter 26 and a magnifying optical unit 27. The beam, and the endothelium image conveyed therewith, joins the central channel 15 in a point where a dichroic mirror 28 is arranged.

The central channel 15 also provides for, starting from the examined eye 7, the above mentioned semireflecting mirror 12 and a central optical unit 29 that concentrates the image of the eye 7 and of the LED 8 to the high speed camera 2, passing through the dichroic mirror 28.

The system is controlled by two pulses 30 and 31 coming from the controller 4. The first pulse 30 transmits the on/off signal to the LEDs 8 and 9, to the photoflash 20 and to the halogen lamp 16. The second pulse 31 transmits the signal for the operation of the motors 6.

The optical head is driven by the motors along three Cartesian directions where the low-high direction corresponds to a Y- direction, the direction of horizontally approaching to and mowing away from the eye corresponds to a Z- direction, and the transversal sideways direction corresponds to a X- direction.

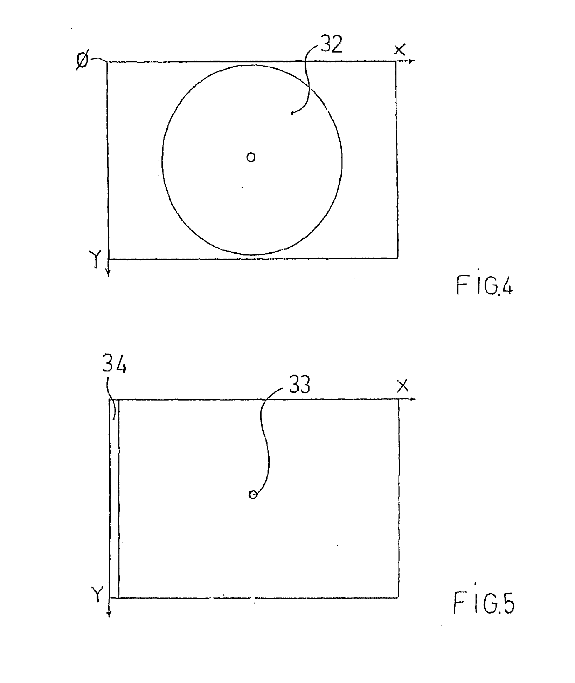

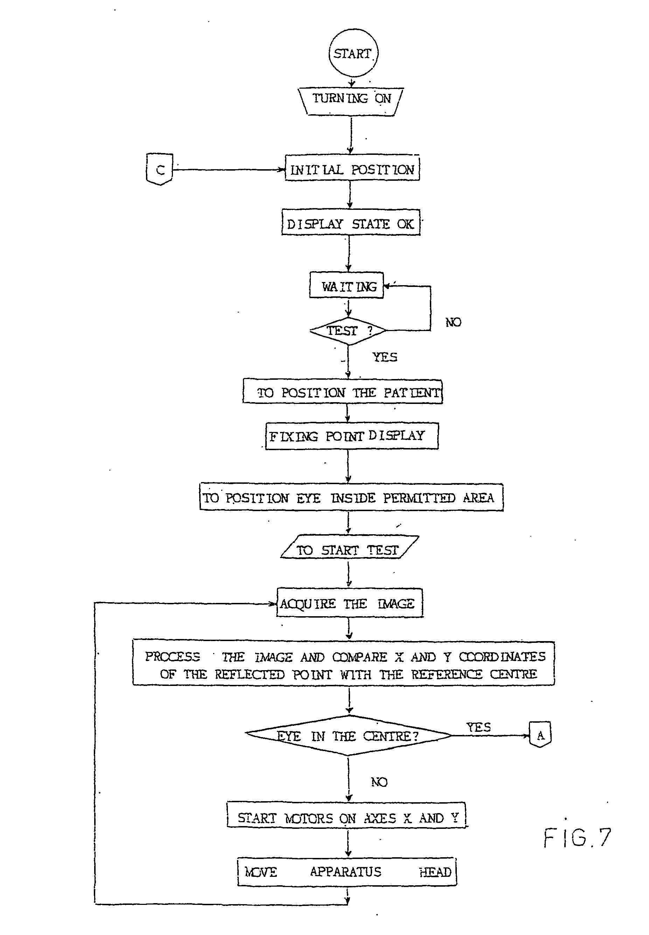

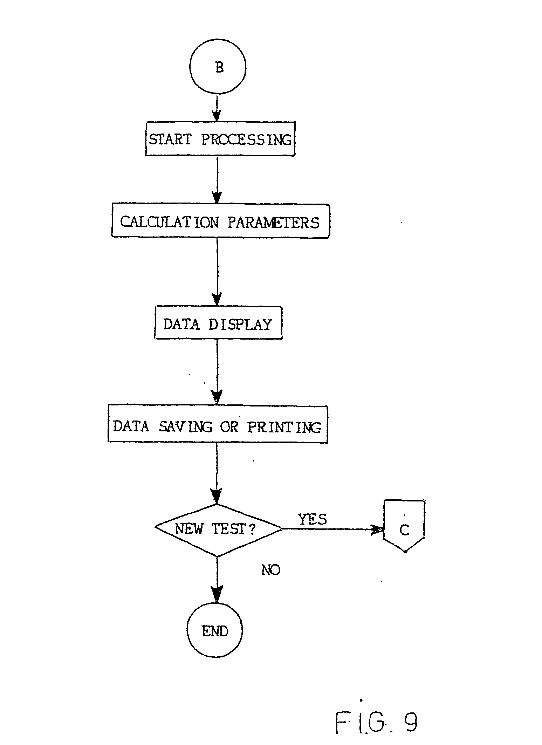

With reference also to figures 4 to 6 and to the self-explanatory flowcharts of figures 7 to 9, the microscope according to the invention works in the following way. After arranging the optical head at the desired position, the test starts with the turning on of LED 9 giving the fixation point for the patient. At the same time, the infrared LED 8 is switched on, projecting via the reflecting mirror 12 a spot of light onto the cornea surface. This spot is detected by the camera 2 along the central channel 15. Camera 2 starts then acquiring images, with a resolution of at least 656 x 400 pixels, taken continuously with a frequency of about 100 Hz.

On each acquired frame, data acquisition procedures are carried out for identifying the points (pixels) in which the grey level is inside a certain predetermined range, so as to eliminate the darker and the clearer points of the prefixed range, and to identify all the points that belong to the light spot reflected by the cornea, and thus to precisely outline the same spot.

Of all the pixels that form the image of the reflected spot the X- and Y- coordinates are calculated, with reference to the upper left angle of the image that coincides with the same position on the sensor of the camera 2 (point ø in figure 4).

Subsequently, average, variance and standard deviation of the X-, Y- coordinates are calculated so as to define the center of the reflected spot and to identify the interference of possible remote luminous signals that could be mistakenly associated with the spot.

The driver board 5 is continuously operated to make the luminous spot given by the LED 8 coincide with the center of the sensor of the camera 2, as a result of the action of the electric motors 6. In practice, the apparatus according to the invention makes the center position of the eye 7 coincide with the center of the CCD sensor of the camera and of the video signal processed by the FireWire IEEE 1394 port and the controller 4, with a feedback control loop to automatically drive the electric motors 6.

In greater detail, the CPU unit 3 determines two concentric areas 32 and 33 (see figures 4 and 5). A bigger area 32 is the area of the image useful to the test, the borders of the image being discarded due to the fact that they are often affected by undesired external reflections. When the center of the above mentioned light spot is outside the area 32, the continuation of the test is not permitted. The area 2 can be circular, as in the example, or shaped differently (oval, squared etc.)

The radius of the area 32 may be defined by the medical operator, or established as a design parameter, the center coinciding with the CCD camera sensor center. A smaller area 33 is instead the optimal area for the centering, i.e. the target area to be reached by the center of the spot in order to deem the eye 7 and camera sensor centered with respect to each other.

After that the center of the reflected spot has been calculated as mentioned, the distance of this from the center of the smaller area 33 (which can even be a single pixel), and the motors are continuously operated to drive the optical head 1 along the X- and Y- directions until such distance is minimized, that is to say the center of the reflected spot is brought (and kept) inside the area 33. In practice, the system automation is therefore to calculate the center position of the reflected spot with respect to the area center 33 so as to instruct the motors accordingly. In this way, through the driver board 5 and the motors 6 placed on two X- Y- directions, the movement of the optical head is driven with a frequency equal to that with which the frames are taken, i.e. every ten milliseconds.

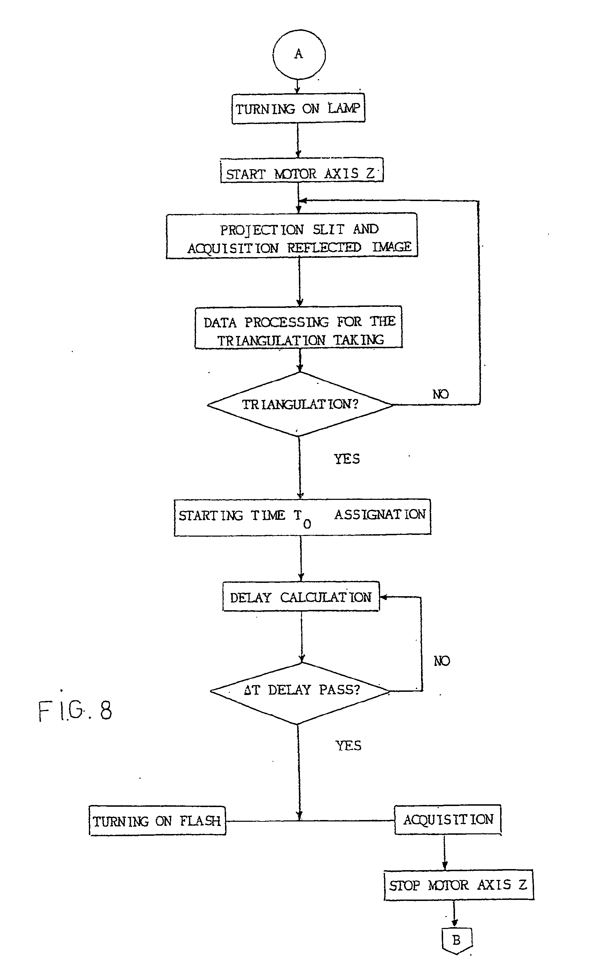

When the reflected image (spot) is deemed centered to the sensor (step A in figures 7 and 8), through a suitable TTL signal that activates the driver board 5, the lamp 16 is switched on. Said lamp 16 illuminates the slit 18 through the lamp condenser 17. The luminous slit that is formed is projected on the eye along the axis 13 through the mirror 19 and the lens 23. The optical head is now moved along the Z- direction, until the triangulation takes place, i.e. until the luminous slit, due to the geometric conditions that regulate the optical reflection, can be reflected by the corneal surface via the reflection axis 14. When this reflection occurs, the image of the slit becomes superimposed to the image acquired by the camera 2 coming from the central channel 15. The same geometric conditions just mentioned are such that the advancement of the optical head along the Z- direction corresponds to a shifting, from the left towards the right (considering the camera sensor as seen in figures 4 and 5) of the image of the slit reflected by the cornea.

In order to have high quality images of the endothelium, it is important that the images be captured, and also (preferably) the cornea be illuminated by the photoflash 20, in the time in which the incident beam coming from the side projection axis 13 is in the optimal position to create the necessary reflection on the layer of the endothelium cells. To this purpose, the apparatus according to the invention proceeds in the following manner.

A check area or band 34 (figure 5) is established on the image taken by the CCD camera sensor, in the left part thereof. In the example the check area 34 is a five pixels wide band starting from the left border of the sensor, but it may be less displaced with respect to the center, and be less wide and long according to the circumstances. In the absence of a triangulation, the image in the check band 34 is generally composed by a grey background with a low intensity value.

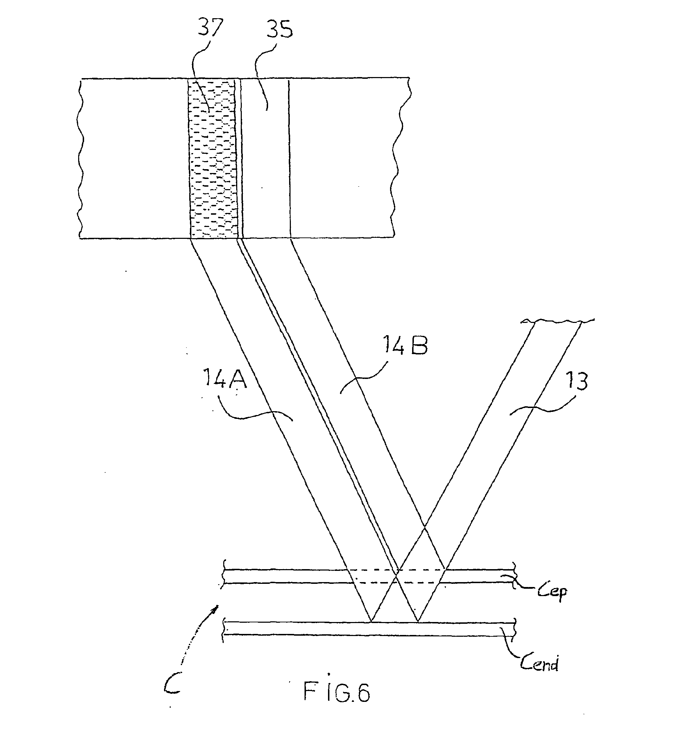

The area 34 is constantly checked, during the advancement along the -Z direction, with the maximum frequency allowed by the characteristics of the camera (for example around 100 frames per second). With particular reference also to figure 6, there is represented a beam 14B reflected by the cornea C, and more precisely by the superficial part thereof, the epithelium Cep. The reflected beam 14B is captured by the camera as a luminous strip 35 (the above mentioned image of the slit) moving from left to right.

When the luminous strip 35 enters the check area 34 the grey level intensity detected therein increases to a bigger value than a predetermined threshold value; this time t₀ is fixed like a temporal reference. The grey level intensity detection in the check area is carried out by average calculations over all the pixels forming the area.

From the time t₀ a suitable delay Δt is set to control the acquisition. In fact, considering the advancement speed of the head along the Z- direction and above all the thickness of the cornea, it is only with a certain delay after the image 35 reflected by the epithelium Cep has been detected in the check area 34, that an image reflected by the endothelium comes to an optimal position for being taken by the camera 2. This situation is clearly represented in the same figure 6, where the beam 14A reflected by the endothelium Cend produces a strip image 37 which is displaced rearward with respect to the image 35 reflected by the epithelium Cep.

The period of time Δt that passes between t₀ (reference) and the time in which the image of the endothelium is taken is then fundamental, and is evaluated on the basis of the advancement speed and the average thickness of the human cornea. The delay time Δt can in any case be adjusted manually or automatically. As the delay time Δt passes, the photoflash 20 is turned on, illuminating the cornea, and the image of the endothelium is taken through the camera 2. A number of different images can also be taken, so that the one having the best quality can be chosen. The images are stored in a database for possible further processing or treatment. As the acquisition cycle is closed, the apparatus returns in the start configuration awaiting a new test to be done.

As mentioned, both the Δt delay and the position of the check area 34 can be changed so as to give to the medical operator the possibility to obtain better images also in case of corneas with particular morphologies. The photoflash lamp 20, thanks to its supplementary luminous impulse, permits to lower the gain of the camera 2 and so to have less noisy images. Said photoflash can be activated with a certain advance with respect to the lapse of Δt, considering the intrinsical lag of the device.

The advantageous characteristics of the apparatus according to the invention attain the object stated in the introductory part. The absence of a photosensor or of a linear sensor along an optical reflection path; the acquisition procedure, controlled and realized by means of simple software instructions given to the apparatus as described above, ensures a better reliability, lower costs and a better use flexibility. Furthermore, the quality of the endothelium images can be increased even further, with respect to known apparatus using conventional focusing techniques, by the possibility of taking a number of frames, and then choosing the highest quality one.

The patients, the tests and the captured images are stored in a database, permitting to work on the taken data even after the test. This permits to rely on useful clinical parameters and, subsequently, to process the same so as to define the number and the density of the cells, their shape, their surface, i.e. their maximum, minimum and average area, the deviation from the standard parameters, a variance coefficient, the ratio of cells of various form, graphics of the distribution, of the dimension of cells areas and graphics of the perimeters distribution. The test can be carried out with a reduced assistance by the medical operator, thanks to the automatic control of the same test as described above.

Variations and/or modifications can be brought to the endothelium reflection microscope for morphometric analysis with direct image acquisition according to the invention, without for this reason departing from the scope of the invention itself as defined in the annexed claims.

## Claims

1. A method for operating an endothelium reflection microscope apparatus, the apparatus comprising an optical head (1) comprising: an illuminating system (16, 17, 18, 36), for obliquely illuminating along a side projection axis (13) through a slit (18) an eyeball surface of a subject eye (7); an eye-front observation optical system (12, 29, 2) along a central channel (15) in which alignment-use indicator light (8) for positional adjustment of the imaging optical center is projected towards the eye (7) and the resulting reflected light spot is received and imaged by a camera (2) comprising a digital optical sensor; and an enlarged-imaging optical system (24, 25, 26, 27) arranged along a side reflection axis (14) for enlarged observation or photographing of the subject part by said digital camera (2) based on slit illuminating light with which the eyeball surface has been illuminated; the apparatus further comprising drive means (6) for moving the optical head (1) along three Cartesian directions comprising an advancement direction (Z-) parallel to said central channel (15) and transverse alignment directions (X-, Y-), and CPU control means (3) for automatically controlling said drive means (6), said illuminating system and said eye-front optical system; the method comprising an alignment procedure in which said optical head (1) is moved along said alignment directions (X-, Y-) so that said reflected light spot and said camera optical sensor are mutually centered, and an endothelium image acquisition procedure in which said optical head (1) is moved along said advancement direction (Z-), the endothelium image acquisition procedure being **characterized in that** it comprises the following steps:
constantly checking the grey level inside a check area (34) of said camera sensor, during the advancement along the advancement direction (Z-), said check area (34) being displaced towards the border of the sensor corresponding to the entry side of the reflection of said slit light, shifting in response to said movement of the optical head along the advancement direction (Z-);
when said grey level reaches a predetermined threshold value, triggering a delay time (Δt); and
when said delay time (At) lapses, enabling the acquisition of one or more images of the endothelium by the digital camera (2).

2. The method according to claim 1, wherein said check area is a strip or band extending vertically for at least a central portion of the sensor.

3. The method according to claim 1 or 2, wherein said grey level is checked by average calculations over all the pixels included in said check area (34).

4. The method according to any of the previous claims, wherein said delay time (At) controls the turning on of supplementary illumination means (20) arranged on said side projection axis (13).

5. The method according to claim 4, wherein said supplementary illumination means comprise a photoflash (20), a turning on signal being emitted from said CPU unit (3) to said photoflash with a certain advance with respect to the lapse of said delay time (At).

6. The method according to any of the previous claims, wherein a plurality of images of the endothelium are taken and the higher quality one is then chosen.

7. The method according to any of the previous claims, wherein said alignment procedure comprises the following steps:
continuously taking images of said reflected light spot;
on each acquired frame, identify all the pixels in which the grey level is inside a certain predetermined range, so as to identify all the points that belong to the light spot reflected by the cornea;
evaluating the center of said light spot;
assessing the distance between the center of said light spot and the center of said sensor;
changing the position of said optical head via said drive motors (6) until said distance is below a predetermined value; and
triggering the start of said endothelium image acquisition procedure.

8. The method according to claim 7, wherein the procedure is stopped or does not start if the center of said light spot is outside of a first, larger control area (32) having its center on the center of said sensor and outlined so as to discard the borders of the sensor.

9. The method according to claim 8, wherein the start of said endothelium image acquisition procedure is triggered when the center of said reflected light spot is inside a second, smaller control area (33) having its center on the center of said sensor.

10. The method according to claim 9, wherein said smaller control area (33) consists in a single pixel.

11. The method according to any of the claims 7 to 10, wherein the center of said reflected light spot is evaluated by calculating the average, variance and standard deviation of the coordinates (X-, Y-) of all the pixels in the reflected light spot, with reference to a fixed reference point of the camera sensor.

12. An endothelium reflection microscope apparatus comprising an optical head (1) comprising: an illuminating system (16, 17, 18, 36), for obliquely illuminating along a side projection axis (13) through a slit (18) an eyeball surface of a subject eye (7); an eye-front observation optical system (12, 29, 2) along a central channel (15) in which alignment-use indicator light (8) for positional adjustment of the imaging optical center is projected towards the eye (7) and the resulting reflected light spot is received and imaged by a camera (2) comprising a digital optical sensor; and an enlarged-imaging optical system (24, 25, 26, 27) arranged along a side reflection axis (14) for enlarged observation or photographing of the subject part by said digital camera (2) based on slit illuminating light with which the eyeball surface has been illuminated; the apparatus further comprising drive means (6) for moving the optical head (1) along three Cartesian directions comprising an advancement direction (Z-) parallel to said central channel (15) and transverse alignment directions (X-, Y-), and CPU control means (3) for automatically controlling said drive means (6), said illuminating system and said eye-front optical system; the CPU control unit being **characterized in that** it is implemented with software instructions for carrying out the method according to any of the previous claims.

## Patentansprüche

1. Verfahren zum Betreiben eines Endothelium-Reflexionsmikroskopgerätes, wobei das Gerät einen optischen Kopf (1) enthält, der enthält: ein Beleuchtungssystem (16, 17, 18, 36), um eine Augapfeloberfläche eines zu testenden Auges (7) schräg längs einer Seitenprojektionsachse (13) durch einen Spalt (18) zu beleuchten; ein optisches Beobachtungssystem (12, 29, 2) für die Augenfront längs eines zentralen Kanals (15), in dem Indikatorlicht (8) zur Ausrichtungsverwendung zur positionsmäßigen Einstellung des optischen Abbildungszentrums zu dem Auge (7) hin projiziert wird und der resultierende reflektierte Lichtfleck empfangen und von einer Kamera (2) abgebildet wird, die einen digitalen optischen Sensor enthält; und ein vergrößert abbildendes optisches System (24, 25, 26, 27), das längs einer Seitenreflexionsachse (14) zur vergrößerten Beobachtung oder zum vergrößerten Fotografieren des zu testenden Teils durch die Digitalkamera (2) basierend auf Spaltbeleuchtungslicht angeordnet ist, mit dem die Augapfeloberfläche beleuchtet wurde, wobei das Gerät ferner Antriebseinrichtungen (6) zum Bewegen des optischen Kopfes (1) längs drei kartesischen Richtungen, die eine Vorwärtsrichtung (Z-) parallel zu dem zentralen Kanal (15) und querverlaufende Ausrichtrichtungen (X-, Y-) enthalten, und CPU-Steuereinrichtungen (3) zum automatischen Steuern der Antriebseinrichtungen (6), der des Beleuchtungssystems und des optischen Systems für die Augenfront enthält; wobei das Verfahren eine Ausrichtprozedur, bei der der optische Kopf (1) längs den Ausrichtrichtungen (X-, Y-) bewegt wird, so dass der reflektierte Lichtfleck und der optische Sensor der Kamera gegenseitig zentriert sind, und eine Endotheliumbild-Erlangungsprozedur enthält, bei der der optische Kopf (1) längs der Vorwärtsrichtung (Z-) bewegt wird, wobei die Endotheliumbild-Erlangungsprozedur **dadurch gekennzeichnet** ist, dass sie die folgenden Schritte enthält:
konstantes Prüfen des Graulevels innerhalb eines Prüfbereichs (34) des Kamerasensors während dem Vorrücken in der Vorwärtsrichtung (Z-), wobei der Prüfbereichs (34), der entsprechend der Eintrittsseite der Reflexion des Spaltlichtes zum Rand des Sensors versetzt wird, sich in Abhängigkeit von der Bewegung des optischen Kopfes längs der Vorwärtsrichtung (Z-) verschiebt;
Triggern einer Verzögerungszeit (Δt), wenn das Graulevel einen vorgegebenen Schwellenwert erreicht; und
Ermöglichen des Erlangens von einem oder mehreren Bild(ern) des Endotheliums durch die Digitalkamera (2), wenn die Verzögerungszeit (Δt) abläuft.

2. Verfahren nach Anspruch 1, wobei der Prüfbereich ein Streifen oder ein Band ist, der wenigstens über einen zentralen Teil des Sensors vertikal verläuft.

3. Verfahren nach Anspruch 1 oder 2, wobei das Graulevel durch Durchschnittsberechnungen über alle Pixel geprüft wird, die in dem Prüfbereich (34) enthalten sind.

4. Verfahren nach einem der vorhergehenden Ansprüche, wobei die Verzögerungszeit (Δt) das Einschalten von Hilfsbeleuchtungseinrichtungen (20) steuert, die an der Seitenprojektionsachse (13) angeordnet sind.

5. Verfahren nach Anspruch 4, wobei die Hilfsbeleuchtungseinrichtungen einen Fotoblitz (20) enthalten, wobei ein Einschaltsignal von der CPU-Einheit (3) zu dem Fotoblitz mit einem bestimmten Vorlauf bezüglich dem Ablauf der Verzögerungszeit (Δt) ausgegeben wird.

6. Verfahren nach einem der vorhergehenden Ansprüche, wobei eine Mehrzahl von Bildern des Endotheliums aufgenommen und dann dasjenige mit der höheren Qualität ausgewählt wird.

7. Verfahren nach einem der vorhergehenden Ansprüche, wobei die Ausrichtprozedur die folgenden Schritte enthält:
kontinuierliches Aufnehmen von Bildern des reflektierten Lichtfleckes;
Identifizieren all der Pixel, in denen das Graulevel innerhalb eines bestimmten vorgegebenen Bereiches ist, an jedem erhaltenen Einzelbild, um all die Punkte zu identifizieren, die zu dem Lichtfleck gehören, der von der Kornea reflektiert wurden;
Evaluieren des Zentrums des Lichtfleckes;
Feststellen des Abstandes zwischen dem Zentrum des Lichtfleckes und dem Zentrum des Sensors;
Ändern der Position des optischen Kopfes über die Antriebsmotoren (6), bis der Abstand unter einem vorgegebenen Wert ist; und
Triggern des Starts der Endotheliumbild-Erlangungsprozedur.

8. Verfahren nach Anspruch 7, wobei die Prozedur angehalten wird oder nicht startet, wenn das Zentrum des Lichtfleckes außerhalb eines ersten größeren Steuerbereiches (32) ist, der sein Zentrum auf dem Zentrum des Sensors und abgegrenzt hat, so dass er die Ränder des Sensors verlässt.

9. Verfahren nach Anspruch 8, wobei der Start der Endotheliumbild-Erlangungsprozedur getriggert wird, wenn das Zentrum des Lichtfleckes innerhalb eines zweiten kleineren Steuerbereiches (33) ist, der sein Zentrum auf dem Zentrum des Sensors hat.

10. Verfahren nach Anspruch 9, wobei der kleinere Steuerbereiches (33) aus einem einzelnen Pixel besteht.

11. Verfahren nach einem der Ansprüche 7 bis 10, wobei das Zentrum des reflektierten Lichtfleckes durch Berechnung der Durchschnitts-, Varianz- und Standardabweichung der Koordinaten (X-, Y-) von allen Pixeln in dem reflektierten Lichtfleck bezüglich eines festen Referenzpunktes des Kamerasensors evaluiert wird.

12. Endothelium-Reflexionsmikroskopgerät, das einen optischen Kopf (1) enthält, enthaltend: ein Beleuchtungssystem (16, 17, 18, 36), um eine Augapfeloberfläche eines zu testenden Auges (7) schräg längs einer Seitenprojektionsachse (13) durch einen Spalt (18) zu beleuchten; ein optisches Beobachtungssystem (12, 29, 2) für die Augenfront längs eines zentralen Kanals (15), in dem Indikatorlicht (8) zur Ausrichtungsverwendung zur positionsmäßigen Einstellung des optischen Abbildungszentrums zu dem Auge (7) hin projiziert wird und der resultierende reflektierte Lichtfleck empfangen und von einer Kamera (2) abgebildet wird, die einen digitalen optischen Sensor enthält; und ein vergrößert abbildendes optisches System (24, 25, 26, 27), das längs einer Seitenreflexionsachse (14) zur vergrößerten Beobachtung oder zum vergrößerten Fotografieren des zu testenden Teils durch die Digitalkamera (2) basierend auf Spaltbeleuchtungslicht angeordnet ist, mit dem die Augapfeloberfläche beleuchtet wurde, wobei das Gerät ferner Antriebseinrichtungen (6) zum Bewegen des optischen Kopfes (1) längs drei kartesischen Richtungen, die eine Vorwärtsrichtung (Z-) parallel zu dem zentralen Kanal (15) und querverlaufende Ausrichtrichtungen (X-, Y-) enthalten, und CPU-Steuereinrichtungen (3) zum automatischen Steuern der Antriebseinrichtungen (6), des Beleuchtungssystems und des optischen Systems für die Augenfront enthält; wobei die CPU-Steuereinheit **dadurch gekennzeichnet** ist, dass sie mit Steuerbefehlen zum Ausführen des Verfahrens nach einem der vorhergehenden Ansprüche ausgestattet ist.

## Revendications

1. Un procédé pour faire fonctionner un appareil microscope par réflexion sur l'endothélium, l'appareil comprenant une tête optique (1) comprenant: un système d'éclairage (16, 17, 18, 36) pour éclairer obliquement, le long d'un axe de projection latéral (13) à travers une fente (18), la surface du globe oculaire d'un oeil (7) d'un sujet ; un système optique d'observation de la face avant d'un oeil (12, 29, 2) suivant un canal central (15) dans lequel une lumière d'indication d'alignement d'utilisation (8) pour le réglage de position du centre optique image est projetée vers l'oeil (7) et la tache lumineuse réfléchie résultante est reçue et convertie en image par un appareil photographique (2) comprenant un capteur optique numérique ; et un système optique d'agrandissement d'image (24, 25, 26, 27) disposé le long d'un axe de réflexion latéral (14) pour une observation agrandie ou pour photographier la partie du sujet à l'aide du dit appareil photographique numérique (2) grâce à la lumière d'éclairage par fente avec laquelle la surface du globe oculaire a été illuminée ; l'appareil comprenant en outre des moyens de déplacement (6) pour déplacer la tête optique (1) le long de trois directions cartésiennes comprenant une direction d'avancement (Z-) parallèle au dit canal central (15) et des directions d'alignement transversales (X-, Y-), et des moyens de commande CPU (3) pour commander automatiquement lesdits moyens de déplacement (6), ledit système d'illumination et ledit système optique pour la face avant de l'oeil ; le procédé comprenant une procédure d'alignement dans laquelle ladite tête optique (1) est déplacée le long des dites directions d'alignement (X-, Y-) de façon que ledit spot de lumière réfléchie et ledit capteur optique de l'appareil photographique soient mutuellement centrés, et une procédure d'acquisition d'images de l'endothélium dans laquelle ladite tête optique (1) est déplacée le long de ladite direction d'avancement (Z-), la procédure d'acquisition d'images de l'endothélium étant **caractérisée en ce qu'**elle comprend les étapes suivantes :
• vérifier constamment le niveau de gris à l'intérieur d'une zone de vérification (34) du dit capteur d'appareil photographique, au cours de l'avancement le long de la direction d'avancement (Z-), ladite zone de vérification (34) étant déplacée vers le bord du capteur correspondant au côté d'entrée de la réflexion de ladite lumière de fente, en la déplaçant en réponse au dit mouvement de la tête optique le long de la direction d'avancement (Z-) ;
• lorsque ledit niveau de gris atteint une valeur de seuil prédéterminée, déclencher un temps de retard (Δt) ; et
• lorsque ledit temps de retard (Δt) est écoulé, permettre l'acquisition d'une ou plusieurs images de l'endothélium par l'appareil photographique numérique (2).

2. Le procédé selon la revendication 1, dans lequel ladite zone de vérification est une bande s'étendant verticalement depuis au moins une partie centrale du détecteur.

3. Le procédé selon l'une des revendications 1 et 2, dans lequel ledit niveau de gris est vérifié par calculs de moyenne sur tous les pixels inclus dans ladite zone de vérification (34).

4. Le procédé selon l'une quelconque des revendications précédentes, dans lequel ledit temps de retard (Δt) commande l'allumage de moyens d'éclairage supplémentaire (20) disposés sur ledit axe de projection latéral (13).

5. Le procédé selon la revendication 4, dans lequel lesdits moyens d'éclairage supplémentaire comprennent un flash lumineux (20), un signal d'allumage étant émis à partir de ladite unité CPU (3) vers ledit flash lumineux avec une certaine avance par rapport à la durée du dit temps de retard (Δt).

6. Le procédé selon l'une quelconque des revendications précédentes, dans lequel une pluralité d'images de l'endothélium sont prises et celle de plus grande qualité est alors choisie.

7. Le procédé selon l'une quelconque des revendications précédentes, dans lequel ladite procédure d'alignement comprend les étapes suivantes:
• prendre en continu des images de ladite tache lumineuse réfléchie;
• sur chaque trame obtenue, identifier tous les pixels dont le niveau de gris est compris dans une certaine fourchette prédéterminée, afin d'identifier tous les points qui appartiennent à la tache lumineuse réfléchie par la cornée;
• déterminer le centre de ladite tache lumineuse;
• estimer la distance entre le centre du dit spot lumineux et le centre du dit capteur;
• changer la position de ladite tête optique par l'intermédiaire des dits moteurs de déplacement (6) jusqu'à ce que ladite distance soit inférieure à une valeur prédéterminée; et
• déclencher le début de ladite procédure d'acquisition d'images de l'endothélium.

8. Le procédé selon la revendication 7, dans lequel la procédure est interrompue ou ne démarre pas si le centre de ladite tache lumineuse se trouve en dehors d'une première, plus grande surface de vérification (32) ayant son centre sur le centre du dit capteur et délimitée de façon à s'écarter des bords du capteur.

9. Le procédé selon la revendication 8, dans lequel le début de ladite procédure d'acquisition d'images de l'endothélium est déclenché lorsque le centre de ladite tache lumineuse réfléchie est à l'intérieur d'une seconde, plus petite surface de vérification (33) ayant son centre sur le centre du dit capteur.

10. Le procédé selon la revendication 9, dans lequel ladite petite surface de vérification (33) est constituée d'un seul pixel.

11. Le procédé selon l'une quelconque des revendications 7 à 10, dans lequel le centre de ladite tache lumineuse réfléchie est déterminé en calculant la moyenne, la variance et l'écart type des coordonnées (X-, Y-) de tous les pixels dans la tache lumineuse réfléchie, par rapport à un point de référence fixe du capteur de l'appareil photographique.

12. Un appareil microscope par réflexion sur l'endothélium comprenant une tête optique (1) comprenant: un système d'éclairage (16, 17, 18, 36) pour éclairer obliquement, le long d'un axe de projection latéral (13) à travers une fente (18), la surface du globe oculaire d'un oeil (7) d'un sujet ; un système optique d'observation de la face avant d'un oeil (12, 29, 2) suivant un canal central (15) dans lequel une lumière d'indication d'alignement d'utilisation (8) pour le réglage de position du centre optique image est projetée vers l'oeil (7) et la tache lumineuse réfléchie résultante est reçue et convertie en image par un appareil photographique (2) comprenant un capteur optique numérique ; et un système optique d'agrandissement d'image (24, 25, 26, 27) disposé le long d'un axe de réflexion latéral (14) pour une observation agrandie ou pour photographier la partie du sujet à l'aide du dit appareil photographique numérique (2) grâce à la lumière d'éclairage par fente avec laquelle la surface du globe oculaire a été illuminée ; l'appareil comprenant en outre des moyens de déplacement (6) pour déplacer la tête optique (1) le long de trois directions cartésiennes comprenant une direction d'avancement (Z-) parallèle au dit canal central (15) et des directions d'alignement transversales (X-, Y-), et des moyens de commande CPU (3) pour commander automatiquement lesdits moyens de déplacement (6), ledit système d'illumination et ledit système optique pour la face avant de l'oeil ; ladite unité de commande CPU étant **caractérisée en ce qu'**elle fonctionne à l'aide d'instructions de logiciel permettant de mettre en oeuvre le procédé selon l'une quelconque des revendications précédentes.
